# EUROPEAN PATENT APPLICATION

(11) **EP 4 556 485 A1**
(43) Date of publication of application: **21.05.2025**
(21) Application number: 23857741.5
(22) Date of filing: 24.08.2023
(51) Int. Cl.: C07K 14/37, C12N 15/70, A23K 20/147, C12N 9/24, A23L 33/18, D21H 17/00, D21H 17/22

(54) **NOVEL POLYPEPTIDE WITH GLUCANASE ACTIVITY**

(30) Priority: 24.08.2022 KR 20220106310
(71) Applicant: CJ CheilJedang Corporation, Seoul 04560 (KR)
(72) Inventor: SON, Byung-sam, Seoul 04560 (KR); CHOI, Eun Jung, Seoul 04560 (KR); SHIM, Jihyun, Seoul 04560 (KR)
(74) Representative: Meissner Bolte Partnerschaft mbB
(86) International application number: PCT/KR2023/012531
(87) International publication number: WO 2024/043709

(57) **Abstract**

The present application relates to a peptide having glucanase activity.

## Description

### [Technical Field]

The present application relates to a polypeptide having glucanase activity.

### [Background Art]

1,3-1,4-β-Glucan is a polysaccharide constituting the cell walls of various plants, and is particularly abundant in the cell walls of commercially valuable grains such as barley, rye, sorghum, rice, and wheat. However, glucan is an anti-nutritional factor that interferes with animals' digestion and absorption of plant-based feeds. Particularly in the case of monogastric animals, it is difficult to degrade and use glucan due to the absence of glucanase (EC 3.2.1.73), which can degrade such anti-nutritional factors.

Therefore, efficient nutrition intake of livestock may be possible by adding glucanase to feed, and for this purpose, development of a glucanase suitable for feed is required (Murphy P, Bello FD, O'Doherty JV, Arendt EK, Sweeney T, Coffey A (2012) Effects of cereal β-glucans and enzyme inclusion on the porcine gastrointestinal tract microbiota. Anaerobe 18(6):557-565).

### [Disclosure]

### [Technical Problem]

Meanwhile, thermal tolerance that can withstand high-temperature processes is essential in the case of enzymes for feeds.

### [Technical Solution]

It is one object of the present application to provide a polypeptide of SEQ ID NO: 4 having glucanase activity.

It is another object of the present application to provide a polynucleotide encoding the polypeptide of the present application.

It is still another object of the present application to provide a microorganism, including one or more among a polypeptide of SEQ ID NO: 4; a polynucleotide encoding the polypeptide; and a vector containing the polynucleotide.

It is yet another object of the present application to provide a composition for degrading glucan, including one or more among a polypeptide of SEQ ID NO: 4; and a microorganism including one or more among a polypeptide of SEQ ID NO: 4, a polynucleotide encoding the polypeptide, and a vector containing the polynucleotide.

It is even another object of the present application to provide a feed additive composition, including one or more among a polypeptide of SEQ ID NO: 4; and a microorganism including one or more among a polypeptide of SEQ ID NO: 4, a polynucleotide encoding the polypeptide, and a vector containing the polynucleotide.

It is further another object of the present application to provide a method for degrading glucan, including: reacting one or more among a polypeptide of SEQ ID NO: 4; and a microorganism including one or more among a polypeptide of SEQ ID NO: 4, a polynucleotide encoding the polypeptide, and a vector containing the polynucleotide with glucan.

It is still further another object of the present application to provide a method for preparing feed products, including: mixing a feed additive composition including one or more among a polypeptide of SEQ ID NO: 4; and a microorganism including one or more among a polypeptide of SEQ ID NO: 4, a polynucleotide encoding the polypeptide, and a vector containing the polynucleotide with a feed component.

It is still further another object of the present application to provide a food composition; a composition for preparing food; a detergent composition; a fabric composition; and a paper processing composition, including one or more among a polypeptide of SEQ ID NO: 4; and a microorganism including one or more among a polypeptide of SEQ ID NO: 4, a polynucleotide encoding the polypeptide, and a vector containing the polynucleotide.

It is still further another object of the present application to provide a method for preparing a polypeptide having glucanase activity, including culturing a microorganism including one or more among a polypeptide of SEQ ID NO: 4; a polynucleotide encoding the polypeptide; and a vector containing the polynucleotide.

### [Advantageous Effects]

The polypeptide having glucanase activity of the present application has improved thermal tolerance and thermal stability, and thus can be advantageously applied in various industrial fields.

### [Brief Description of the Drawing]

FIG. 1 is a graph confirming the activity of the glucanase reference sequence (SEQ ID NO: 2) derived from the genus *Orpinomyces* and the novel glucanase (SEQ ID NO: 4) of the present application according to pH.
FIG. 2 is a graph confirming the activity of the glucanase reference sequence (SEQ ID NO: 2) derived from the genus *Orpinomyces* and the novel glucanase (SEQ ID NO: 4) of the present application according to temperature.
FIG. 3 is a graph confirming the residual activity of the glucanase reference sequence (SEQ ID NO: 2) derived from the genus *Orpinomyces* and the novel glucanase (SEQ ID NO: 4) of the present application according to temperature change.

### [Detailed Description of Preferred Embodiments]

Hereinafter, the present application will be described in detail. Meanwhile, each description and embodiment disclosed herein can be applied to other descriptions and embodiments, respectively. That is, all combinations of various elements disclosed herein fall within the scope of the present application. Further, the scope of the present application is not limited by the specific description described below.

Additionally, those of ordinary skill in the art may be able to recognize or confirm, using only conventional experimentation, many equivalents to the particular aspects of the invention described herein. Furthermore, it is also intended that these equivalents be included in the present application.

Further, a number of papers and patent documents have been cited throughout the present specification. The content of the cited papers and patent documents is incorporated herein by reference in their entirety and the level of technical field to which the present invention belongs and the contents of the present invention will be described more clearly.

One aspect of the present application provides a polypeptide of SEQ ID NO: 4, having glucanase activity. The polypeptide is a novel polypeptide having glucanase activity.

As used herein, the term "glucanase" means an enzyme having an activity capable of cleaving a glucosidic bond connecting two glucosyl residues in glucan. Non-limiting examples of glucanase activity include endo-cellulase activity for beta-1,4 linkages between D-glucoses, and licheninase or lichenase activity that cleaves (1,4)-beta-D-glucosidic bonds of beta-D-glucans containing 1,3- and 1,4-linkages. As one embodiment, the glucanase having glucanase activity of the present application may have an activity corresponding to glucanase classified as EC 3.2.1.73.

Although the novel polypeptide having glucanase activity provided in the present application has been defined as the polypeptide of SEQ ID NO: 4, it does not exclude an addition of a meaningless sequence upstream or downstream of SEQ ID NO: 4, a mutation that may occur naturally, or a silent mutation thereof, and it is apparent to those skilled in the art that any polypeptide having an activity identical or corresponding to the protein consisting the amino acid sequence of SEQ ID NO: 4 may fall within the scope of the polypeptide having glucanase activity provided in the present application.

That is, although it is described as "a protein or polypeptide having an amino acid sequence described by a particular sequence number" or "a protein or polypeptide including an amino acid sequence described by a particular sequence number" in the present application, it is apparent that any protein having an amino acid sequence in which part of the sequence is deleted, modified, substituted, or added can be used in the present application even if it has the same or corresponding activity as the polypeptide consisting of the amino acid sequence of the corresponding sequence number.

Additionally, a polypeptide having glucanase activity obtained by aligning the sequences of the polypeptide of SEQ ID NO: 4 provided in the present application and the reference glucanase polypeptide and comparing the reference sequence with the sequence of the polypeptide provided in the present application, thereby introducing a modification corresponding to the polypeptide provided in the present application into the reference sequence of the glucanase may also be included in the scope of polypeptides provided in the present application. One example of such a reference sequence may include an amino acid sequence of SEQ ID NO: 2 or a polypeptide having an identity of at least 90%, 91%, 92%, 93%, 94%, 95%, 96%, 97%, 98%, 99%, or more thereto. The reference sequence may be used as a parent sequence for introducing a modification or as a control for evaluating thermal stability and/or thermal tolerance.

As another example of the reference sequence, a wild-type glucanase sequence can be used. Examples of the wild-type glucanase include glucanases derived from *Orpinomyces* sp., *Neocallimastix* sp., *Piromyces* sp., or *Ruminococcus* sp. One example of the wild-type glucanase may include the amino acid sequence of NCBI Sequence ID No. 014412.1 or a polypeptide having an identity of at least 90%, 91%, 92%, 93%, 94%, 95%, 96%, 97%, 98%, 99% or more thereto.

The polypeptide of SEQ ID NO: 4 provided in the present application may be a polypeptide of a wild-type glucanase known in the art, for example, a wild-type glucanase derived from *Orpinomyces* sp., and specifically, it may be referred to a "modified polypeptide" or "variant" as compared to a "wild type" in the way that one or more amino acids are different from the amino acid sequence of NCBI Sequence ID No. 014412.1.

In addition, a modified polypeptide, in which one or more amino acids are different from the recited sequence by conservative substitutions and/or modifications with comparison to the amino acid sequence of SEQ ID NO: 4, while the functions and properties of the protein are retained, may also be included within the scope of the polypeptide provided in the present application. Such a modification may include, for example, a modification in which a region has been removed from the N- and/or C-terminal of a mature protein.

As used herein, the term "conservative substitution" refers to substitution of an amino acid with another amino acid having similar structural and/or chemical properties. The polypeptide of the present application may have, for example, one or more conservative substitutions while still retaining one or more biological activities possessed by the polypeptide of SEQ ID NO: 4. Such amino acid substitution may generally occur based on similarity of polarity, charge, solubility, hydrophobicity, hydrophilicity, and/or amphipathic nature of a residue.

In one embodiment, the novel polypeptide having glucanase activity provided in the present application may include SEQ ID NO: 4, consist essentially of SEQ ID NO: 4, or consist of SEQ ID NO: 4. In another embodiment, the novel polypeptide having glucanase activity provided in the present application may have a homology or identity of at least 80%, 90%, 91%, 92%, 93%, 94%, 95%, 96%, 97%, 98%, 99% or more, or 100% to SEQ ID NO: 4. Additionally, it may include, consist essentially of, or consist of a sequence having the above homologies or identities.

As used herein, the term "homology" or "identity" refers to a degree of relatedness between two given amino acid sequences or nucleotide sequences, and may be expressed as a percentage. The terms "homology" and "identity" may often be used interchangeably with each other.

The sequence homology or identity of conserved polynucleotides or polypeptides may be determined by standard alignment algorithms and can be used with a default gap penalty established by the program being used. Substantially, homologous or identical sequences may generally hybridize under moderately or highly stringent conditions to the full length of the sequence or at least about 50%, 60%, 70%, 80%, or 90% of the full length. It is apparent that hybridization with polynucleotides containing general codon or degenerate codons in hybridizing polynucleotides is also included.

Whether any two polynucleotide or polypeptide sequences have a homology, similarity, or identity may be, for example, determined by a known computer algorithm such as the "FASTA" program (Pearson et al., (1988) Proc. Natl. Acad. Sci. USA 85:2444) using default parameters. Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (preferably, version 5.0.0 or later) (GCG program package (Devereux J et al., Nucleic Acids Research 12:387 (1984)), BLASTP, BLASTN, FASTA (Atschul SF et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity, or identity may be determined using BLAST or ClustalW of the National Center for Biotechnology Information (NCBI).

The homology, similarity, or identity of polynucleotides or polypeptides may be, for example, determined by comparing sequence information using, for example, the GAP computer program, such as Needleman et al. (1970), J Mol Biol. 48:443 as disclosed in Smith and Waterman, Adv. Appl. Math (1981) 2:482. In summary, the GAP program defines the homology, similarity, or identity as the value obtained by dividing the number of similarly aligned symbols (*i.e.,* nucleotides or amino acids) by the total number of the symbols in the shorter of the two sequences. Default parameters for the GAP program may include (1) a binary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide sequences have a homology, similarity or identity with each other may be identified by comparing the sequences in a Southern hybridization experiment under stringent conditions as defined, and appropriate hybridization conditions defined are within the skill of the art, and may be determined by a method well known to those skilled in the art (e.g., J Sambrook et al., Molecular Cloning, A Laboratory Manual, 2nd Edition, Cold Spring Harbor Laboratory press, Cold Spring Harbor, New York, 1989; FM Ausubel et al., Current Protocols in Molecular Biology, John Wiley & Sons, Inc., New York), but is not limited thereto.

As used herein, the "enzymatic activity" or "glucanase activity" exhibits at least one catalytic activity. Specifically, it may be the conversion efficiency of an enzyme mainly expressed as k_{cat}/Kₘ, but is not limited thereto.

When the enzyme is completely saturated with substrates, k_{cat} means the rate constant (catalytic constant) that converts substrates into products in unit time by one enzyme, and is also referred to as a turnover number. Kₘ is the substrate concentration when the reaction rate is at half the maximum value (Vₘₐₓ).

Examples of the ways to express enzymatic activity include specific activity (µmol of converted substrate × mg⁻¹ × min⁻¹) or volumetric activity (µmol of converted substrate × mL⁻¹ × min⁻¹), *etc.* However, defining the enzymatic activity is not limited to the contents described above, and the enzymatic activity can be defined and evaluated based on the information known in the art.

As used herein, enzyme stability means that enzyme activity is maintained during storage or reaction time. In order to measure such changes in stability, the initial enzyme activity can be measured and compared under defined conditions at time zero (100%) and after a certain period of time (x%), and thus, the level at which the enzyme activity is lost or enzyme stability can be expressed.

Factors that affect enzyme activity include, for example, pH, heat, the presence of other substances (*e.g.,* oxidizing agents, chelating agents), *etc.*

As used herein, the term "thermal stability" means the ability of a protein to function in a specific temperature range. In one embodiment, the variant provided in the present application may have activity in the range of about 20°C°C to about 120°C, and specifically may have activity in the range of about 60°C to about 100°C, but is not limited thereto. In any one of the embodiments described above, the polypeptide having glucanase activity provided in the present application may have activity at 50°C to 80°C, but the temperature range is not limited thereto.

As used herein, the term "thermal tolerance" means the ability of a protein to function after exposure to a specific temperature, *e.g.,* high heat or cryogenic temperature. For example, proteins that are thermotolerant may not function at a temperature they are exposed to, but may become functional when returned to an optimum temperature environment.

An increase in stability may include maintaining high enzymatic activity; increasing the range of pH, temperature and/or time, *etc.,* at which a protein maintains its function, by comparing to other enzymes, *e.g.,* a wild-type enzyme, parent enzyme and/or other modified polypeptides. In the present application, the increase in stability may be compared relative to the wild-type glucanase, for example, glucanase of SEQ ID NO: 2, but is not limited thereto.

In one embodiment, the polypeptide having glucanase activity provided in the present application may have one in which one or more properties selected from thermal tolerance and thermal stability are improved compared to the polypeptide of SEQ ID NO: 2.

In any one of the embodiments described above, the variant may maintain a relative activity of at least 20% or more at 50°C to 80°C, but is not limited thereto.

In any one of the embodiments described above, the properties exhibited by the polypeptide having glucanase activity provided in the present application activity may be suitable or improved activity for application in various industrial fields, including feed, baking, pulp bleaching, *etc.*

Another aspect of the present application provides a polynucleotide encoding the polypeptide having glucanase activity of the present application.

As used herein, the "polynucleotide", which is a polymer of nucleotides composed of nucleotide monomers connected in a lengthy chain by a covalently bond, is a DNA or RNA strand having at least a certain length.

The polynucleotide encoding the polypeptide having glucanase activity of the present application may include any polynucleotide encoding the polypeptide of SEQ ID NO: 4 or a polypeptide having activity corresponding thereto, without limitation.

The polynucleotide encoding the polypeptide having glucanase activity of the present application may undergo various modifications in the coding region within the scope that does not change the amino acid sequence of the polypeptide, due to codon degeneracy or in consideration of the codons preferred in an organism in which the polypeptide is to be expressed.

For example, the polynucleotide encoding the polypeptide having glucanase activity of the present application may be the amino acid sequence of SEQ ID NO: 4 or a polynucleotide sequence encoding a polypeptide having a homology or identity thereto.

In one embodiment, the polynucleotide encoding the polypeptide having glucanase activity of the present application may have or include a nucleotide sequence having a homology or identity of at least 50%, 60%, 70%, 75%, 80%, 85% or 90% or more, and less than 100% with the polynucleotide sequence of SEQ ID NO: 1. In another embodiment, the polynucleotide encoding the polypeptide having glucanase activity of the present application may consist of or consist essentially of the sequence of SEQ ID NO: 3 or a nucleotide sequence having a homology or identity of at least 70%, 75%, 80%, 85%, 90%, 95%, 96%, 97%, 98%, or 99% or more to the sequence of SEQ ID NO: 3, but is not limited thereto.

Additionally, the polynucleotide of the present application may include a probe that may be prepared from a known gene sequence, for example, any sequence which can hybridize with a sequence complementary to all or part of the polynucleotide sequence of the present application under stringent conditions without limitation.

The "stringent conditions" refers to conditions under which specific hybridization between polynucleotides is allowed. Such conditions are specifically described in the literature (J Sambrook et al., Molecular Cloning, A Laboratory Manual, 2nd Edition, Cold Spring Harbor Laboratory press, Cold Spring Harbor, New York, 1989; FM Ausubel et al., Current Protocols in Molecular Biology, John Wiley & Sons, Inc., New York, 9.50-9.51, 11.7-11.8). For example, the stringent conditions may include conditions under which genes having a high homology or identity of 70% or more, 75% or more, 80% or more, 85% or more, 90% or more, 95% or more, 96% or more, 97% or more, 98% or more, or 99% or more are hybridized with each other and genes having a homology or identity lower than the above homologies or identities are not hybridized with each other, or washing conditions of Southern hybridization, that is, washing once, specifically, twice or three times at a salt concentration and a temperature corresponding to 60°C, 1×SSC, 0.1% SDS, specifically 60°C, 0.1×SSC, 0.1% SDS, and more specifically 68°C, 0.1×SSC, 0.1% SDS.

Hybridization requires that two nucleic acids contain complementary sequences, although mismatches between bases are possible depending on the stringency of the hybridization. The term "complementary" is used to describe the relationship between nucleotide bases that can hybridize with each other. For example, with respect to DNA, adenine is complementary to thymine, and cytosine is complementary to guanine. Therefore, the polynucleotide of the present application may include isolated nucleotide fragments complementary to the entire sequence as well as nucleic acid sequences substantially similar thereto.

Specifically, polynucleotides having a homology or identity with the polynucleotide of the present application may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (*e.g.,* Sambrook *et al*.).

Still another aspect of the present application provides a vector containing a polynucleotide encoding the polypeptide having glucanase activity of the present application. The peptide and the polynucleotide are as described in other aspects above.

As used herein, the term "vector" may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable microorganism, the vector may replicate or function independently from the host genome, or may integrate into genome thereof.

In one example, a polynucleotide encoding a target protein may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface polypeptides, may be used. Only cells expressing the selection marker are able to survive or to show different phenotypes under the environment treated with the selective agent, and thus the transformed cells may be selected.

The vector used in the present application is not particularly limited, and any vector known in the art may be used.

Examples of the vector typically used in prokaryotic cells may include, as a phage vector or cosmid vector, pWE15, M13, MBL3, MBL4, IXII, ASHII, APII, t10, t11, Charon4A, and Charon21A, *etc.;* and as a plasmid vector, those based on pBR, pUC, pBluescriptII, pGEM, pTZ, pCL and pET, *etc.* Specifically, pDZ, pACYC177, pACYC184, pCL, pECCG117, pUC19, pBR322, pMW118, pCC1BAC, pDCM2 vector, *etc.* may be used.

As examples of the vector used in eukaryotic cells, yeast expression vector may include both yeast integrative plasmid (YIp) and extrachromosomal plasmid vector. The extrachromosomal plasmid vector may include yeast episomal plasmid (YEp), yeast replicative plasmids (YRp), and yeast centromere plasmid (YCp). In addition, yeast artificial chromosomes (YACs) can also be used as the vector of the present application. Specific examples of the vector that can be used may include pESCHIS, pESC-LEU, pESC-TRP, pESC-URA, Gateway pYES-DEST52, pAO815, pGAPZ A, pGAPZ B, pGAPZ C, pGAPα A, pGAPα B, pGAPα C, pPIC3.5K, pPIC6 A, pPIC6 B, pPIC6 C, pPIC6α A, pPIC6α B, pPIC6α C, pPIC9K, pYC2/CT, pYD1 Yeast Display Vector, pYES2, pYES2/CT, pYES2/NT A, pYES2/NT B, pYES2/NT C, pYES2/CT, pYES2.1, pYES-DEST52, pTEF1/Zeo, pFLD1, PichiaPinkTM, p427-TEF, p417-CYC, pGAL-MF, p427-TEF, p417-CYC, PTEF-MF, pBY011, pSGP47, pSGP46, pSGP36, pSGP40, ZM552, pAG303GAL-ccdB, pAG414GAL-ccdB, pAS404, pBridge, pGAD-GH, pGAD T7, pGBK T7, pHIS-2, pOBD2, pRS408, pRS410, pRS418, pRS420, pRS428, yeast micron A form, pRS403, pRS404, pRS405, pRS406, pYJ403, pYJ404, pYJ405 and pYJ406, but are not limited thereto.

As used herein, the term "transformation" refers to the introduction of a vector containing a polynucleotide encoding a target protein into a host cell or a microorganism so that the protein encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target protein. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into a host cell as it is and operably linked to sequences required for expression in the host cell, but is not limited thereto.

Further, as used herein, the term "operably linked" means that the gene sequence is functionally linked to a promoter sequence that initiates and mediates transcription of the polynucleotide encoding the target polypeptide of the present application.

The method of transforming the vector of the present application may include any method which can introduce a nucleic acid into a cell, and the transformation may be performed by selecting an appropriate standard technique as known in the art according to the host cell. For example, the method may include electroporation, calcium phosphate (CaPO₄) precipitation, calcium chloride (CaCl₂) precipitation, microinjection, a polyethylene glycol (PEG) method, a DEAE-dextran method, a cationic liposome method, and a lithium acetate-DMSO method, *etc.,* but is not limited thereto.

Yet another aspect of the present application provides a microorganism including one or more among the polypeptide having glucanase activity of the present application; a polynucleotide encoding the polypeptide; and a vector containing the polynucleotide.

The microorganism may include the above-described polypeptide, a polynucleotide encoding the polypeptide, a nucleic acid construct and/or vector containing the polynucleotide. The nucleic acid construct or vector may be integrated into the chromosome, or may remain as a self-replicating extrachromosomal vector.

The microorganism of the present application may include any microorganism as long as it can express the polypeptide having glucanase activity of the present application without limitation. For example, the microorganism may include any cell useful in recombinant production of the polypeptide having glucanase activity of the present application, *e.g.,* a prokaryote or a eukaryote. In another example, the microorganism includes fungi and bacteria.

In one embodiment, the microorganism of the present application may be a microorganism belonging to the genus *Escherichia,* and may be *Escherichia coli, Escherichia albertii, Escherichia fergusonii, Escherichia hermannii, Escherichia vulneris,* or *Escherichia blattae.* In any one of the embodiments described above, the microorganism may be *Escherichia coli,* but is not limited thereto.

Even another aspect of the present application provides a composition for degrading glucan, including one or more among the polypeptide having glucanase activity of the present application; and a microorganism including one or more among the polypeptide, a polynucleotide encoding the polypeptide, and a vector containing the polynucleotide.

Even another aspect of the present application provides a use of a composition for degrading glucan, wherein the composition includes one or more among the polypeptide having glucanase activity of the present application; and a microorganism including one or more among the polypeptide, a polynucleotide encoding the polypeptide, and a vector containing the polynucleotide.

Further another aspect of the present application provides a method for degrading glucan, including: reacting one or more among the polypeptide having glucanase activity of the present application; and a microorganism including one or more among the polypeptide, a polynucleotide encoding the modified polypeptide, and a vector containing the polynucleotide with glucan.

The polypeptide having glucanase activity of the present application and/or the microorganism including one or more among the polypeptide, a polynucleotide encoding the modified polypeptide, and a vector containing the polynucleotide may be used to degrade a glucan-containing material.

In one example, the composition for degrading glucan provided in the present application may further include a naturally occurring material or a non-naturally occurring material, in addition to the polypeptide of the present application.

In one embodiment, the composition for degrading glucan provided in the present application may further include any constituting elements suitable for application in various industrial fields, including animal feed, baking, biomass saccharification, pulp bleaching, *etc.*

Examples of materials that may be added include stabilizers, surfactants, builders, chelating agents, dispersants, enzymes, enzyme stabilizers, catalysts, activators, carriers, binders, lubricants, disintegrants, excipients, solubilizers, suspending agents, colorants, flavoring agents, buffers, preservatives, analgesics, solubilizers, isotonic agents, *etc.,* but are not limited thereto.

Still further another aspect of the present application provides a feed additive composition, including one or more among the polypeptide having glucanase activity of the present application; and a microorganism including one or more among the polypeptide, a polynucleotide encoding the modified polypeptide, and a vector containing the polynucleotide.

Still further another aspect of the present application provides a method for preparing feed products, including: mixing a feed additive composition including one or more among the polypeptide having glucanase activity of the present application; and a microorganism including one or more among the polypeptide, a polynucleotide encoding the modified polypeptide, and a vector containing the polynucleotide with a feed component.

The microorganism including the polypeptide having glucanase activity of the present application, a polynucleotide encoding the modified polypeptide, and/or a vector containing the polynucleotide may be used in any one of the following applications:
a) an additive in animal feedstuffs; and/or
b) a feed supplement for animals; and/or
c) breakdown of grain-based materials (e.g., this can be whole grain or part of grain).

The feed product of the present application may refer to any natural or artificial diet, a single meal, *etc.,* or a component of the single meal, which an animal eats, ingests and digests, or which is suitable for eating, ingestion and digestion, and the feed product may be prepared in various forms known in the art.

The glucanase of the present application can be used in various industrial fields such as food, paper, pulp, detergent, industrial waste treatment, biomaterials and bioenergy, *etc.* With respect to the application in the industrial fields, literature such as McCarthy TC, Lalor E, Hanniffy O, Savage AV, Tuohy MG, Comparison of wide-type and UV-mutant βglucanase-producing strains of Talaromyces emersonii with potential in brewing applications, J Ind Microbiol Biotechnol, 2005; Chaari F, Belghith-Fendri L, Blibech M, Driss D, Ellouzia SZ, Sameh M, Ellouz-Chaabouni S, Biochemical characterization of a lichenase from Penicillium occitanis Pol6 and its potential application in the brewing industry, Process Biochem, 2014; Maktouf S, Moulis C, Kamoun A, Chaari F, Chaabouni SE, Remaud-Simeon M, A laundry detergent compatible lichenase: statistical optimization for production under solid state fermentation on crude millet, Ind Crops Prod, 2013; *etc.* may be incorporated by reference.

Still further another aspect of the present application provides a food composition; and a composition for preparing food, including the glucanase of the present application or the microorganism of the present application. In one example, glucanase can be used for brewing (beer, wine, *etc*.) and for producing fruit juice. Glucanase can be added during saccharification to reduce viscosity of wort, improve filtration performance, increase the dissolution rate of malt, prevent beer turbidity, and stabilize quality of beer.

Still further another aspect of the present application provides a detergent composition including the glucanase of the present application or the microorganism of the present application. Detergent compositions are compositions used to remove unwanted compounds from items to be cleaned, such as fabrics, dishes, or hard surfaces. Examples of detergent compositions include fabric detergents, textile detergents, hard surface cleaners for glass, wood, plastic, ceramic, metal countertops and windows, *etc.,* carpet cleaners, oven cleaners, fabric fragrances, fabric softeners, dishwasher detergents, *etc.*

Still further another aspect of the present application provides a fabric composition including the glucanase of the present application or the microorganism of the present application. The glucanases of the present application can be used in the processing of fibers for the manufacture of garments. These include processes such as bleaching, dye removal after dyeing, and biopolishing of cotton fabrics or cotton blends.

Still further another aspect of the present application provides a paper processing composition including the glucanase of the present application or the microorganism of the present application. The glucanase of the present application can be used for improving wastewater, removing ink or decolorization in the pulp industry.

In any one of the embodiments described above, the composition provided in the present application may further include any constituting element suitable for application in various industrial fields. In any one of the embodiments described above, the composition provided in this application may further include any other enzyme suitable for the intended use.

In any one of the embodiments described above, the composition provided in the present application may include the glucanase of the present application or the microorganism of the present application in an amount suitable for the intended use.

In any one of the embodiments described above, the composition provided in the present application may be provided in a form suitable for the intended use. As non-limiting examples, the composition may be in the form of bars, tablets, powder, granules, pastes or liquid.

Still further another aspect of the present application provides a method for preparing a polypeptide having glucanase activity, including: culturing a microorganism including one or more among the polypeptide having glucanase activity of the present application, a polynucleotide encoding the polypeptide, and a vector containing the polynucleotide.

The cultivation may include culturing the microorganism in a culture medium.

As used herein, the term "cultivation" means that the host cell is grown under appropriately controlled environmental conditions. The cultivation process of the present application may be performed in a suitable culture medium and culture conditions known in the art. Such a cultivation process may be easily adjusted for use by those skilled in the art according to the strain to be selected. Specifically, the cultivation may be a batch culture, a continuous culture, and a fed-batch culture, but is not limited thereto.

As used herein, the term "medium" refers to a mixture of materials which contains nutrient materials required for the cultivation of the host cell as a main ingredient, and it supplies nutrient materials and growth factors, along with water that is essential for survival and growth. Specifically, the medium and other culture conditions used for culturing the host cell of the present application may be any medium used for conventional cultivation of host cells without any particular limitation. However, the host cell of the present application may be cultured under aerobic conditions in a conventional medium containing an appropriate carbon source, nitrogen source, phosphorus source, inorganic compound, amino acid, and/or vitamin, *etc.,* while adjusting temperature, pH, *etc.*

In the present application, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.* Additionally, the carbon source may include natural organic nutrients such as starch hydrolysate, molasses, blackstrap molasses, rice bran, cassava, sugar cane molasses, and corn steep liquor, *etc.* Specifically, carbohydrates such as glucose and sterilized pretreated molasses (*i.e.,* molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc.;* amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compounds may include sodium chloride, calcium chloride, iron chloride, magnesium sulfate, iron sulfate, manganese sulfate, calcium carbonate, *etc.* Additionally, amino acids, vitamins, and/or appropriate precursors may be included. These constituting ingredients or precursors may be added to a medium in a batch or continuous manner, but these phosphorus sources are not limited thereto.

Additionally, the pH of the medium may be adjusted by adding a compound such as ammonium hydroxide, potassium hydroxide, ammonia, phosphoric acid, sulfuric acid, *etc.* during the cultivation of the host cell in an appropriate manner. In addition, bubble formation may be prevented during the cultivation using an antifoaming agent such as fatty acid polyglycol ester. Further, oxygen gas or a gas containing oxygen may be injected to the medium in order to maintain aerobic conditions of the medium; or nitrogen gas, hydrogen gas, or carbon dioxide may be injected to maintain anaerobic or microaerobic conditions, without the injection of gas, but the gas is not limited thereto.

The temperature of the medium may be in the range from 20°C to 55°C, specifically from 25°C to 40°C, but is not limited thereto. The cultivation may be continued until the desired amount of a useful material is obtained, and may be specifically carried out for 24 to 196 hours, but is not limited thereto.

In one embodiment, the method for preparing the polypeptide having glucanase activity of the present application may further include recovering the polypeptide having glucanase activity of the present application expressed in the culturing step.

In another embodiment, the polypeptide having glucanase activity expressed in the culturing step may be recovered using methods known in the art to which the present application pertains. For example, the polypeptide may be recovered from a medium by conventional procedures including, but not limited to, collection, centrifugation, filtration, extraction, spray-drying, evaporation, or precipitation.

With respect to the recovering method, the polypeptide may be collected using the method of culturing the microorganism of the present application, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, *etc.,* HPLC and a combination thereof may be used, and the polypeptide of the present application may be recovered from the medium or the host cell using suitable methods known in the art.

In another embodiment, the polypeptide expressed by the host cell in the culturing step may not be recovered. In the embodiment, the host cell expressing the polypeptide may be used as a source of the polypeptide.

Still another aspect of the present application provides a composition including one or more among the polypeptide having glucanase activity of the present application; and a microorganism including one or more among the polypeptide, a polynucleotide encoding the polypeptide, and a vector containing the polynucleotide.

The composition can be used for food, feed additive, food preparation, detergent, fabric or paper processing as described above. Still further another aspect of the present application provides a use of the composition for food, feed additive, food preparation, detergent, fabric or paper processing, as described in other aspects above.

### [Mode for Carrying Out the Invention]

Hereinafter, the present application will be described in more detail by way of Examples and Experimental Examples. However, these Examples and Experimental Examples are given for illustrative purposes only, and the scope of the present application is not intended to be limited by these Examples and Experimental Examples.

### Example 1. Preparation of Protein Having Glucanase Activity

### Example 1-1. Preparation of Template

The reference gene corresponding to 1,3-1,4-β-glucanase in the genomic DNA of the *Orpinomyces* sp. PC-2 strain was codon-optimized to be suitable for expression in *E. coli* (SEQ ID NO: 1). In addition, in order to improve thermal tolerance, a variant gene, in which multiple point mutations were introduced based on SEQ ID NO: 1 as a template, was codon-optimized (SEQ ID NO: 3). Finally, the thus-optimized gene was synthesized by Cosmogenetech Co., Ltd.

The reference sequence was used as a control for the experiment, and the synthesized control and the variant gene were each cloned into the pET28a vector (pET28-W, pET28-V) for expression.

### Example 1-2. Preparation of Transformants Having Glucanase Gene and Enzyme Expression

The recombinant plasmids pET28-W and pET28-V were transformed with *E. coli* BL21. In order to produce the control and variant, the transformants were inoculated into 5 mL of LB medium and pre-incubated at 37°C for 18 hours. For the main culture, 5 mL of the pre-culture medium was inoculated into 300 mL of LB medium and incubated at 37°C. Then, when the OD₆₀₀ value reached between 0.4-0.6, 120 µL of 1 M IPTG was added and incubated for an additional 12 hours. Cells were recovered through centrifugation and redispersed in 35 mL of lysis buffer (50 mM Tris-HCl pH 8.0, 100 mM NaCl, 10 mM imidazole).

### Example 1-3. Purification of Protein Having Glucanase Activity

The cells in the dispersed solution were disrupted using an ultrasonic disperser, and a crude enzyme solution, the supernatant, was obtained through centrifugation. The crude enzyme solution was flowed through a Ni-NTA resin (Qiagen, Cat. No. 30230) to be adsorbed, followed by sequentially flowing washing buffer (only 20 mM of imidazole in the lysis buffer composition) and elution buffer (only 250 mM of imidazole in the lysis buffer composition) to obtain purified control and variant.

### Example 2. Evaluation of Properties of Glucanase Variant

### Example 2-1. Measurement of Activity of Glucanase Variant

In order to evaluate the properties of the control and variant, 50 µL of 1% glucan, 5 µL of 1 M buffer, and an appropriate amount of the enzymes were mixed, and tertiary distilled water was added to a final volume of 100 µL. Thereafter, the mixture was stirred well and reacted at 37°C, and the produced reducing sugar was measured by the DNS method. The method for preparing a DNS solution was as follows:
6.3 g of 3,5-dinitrosalicylic acid (samchus, D1267) was added to 500 mL of distilled water and dissolved at 50°C while 21 g of sodium hydroxide (Daejung, 7571-4400) was added. 182 g of potassium sodium tartrate tetrahydrate (Daejung, 6618-4400) and 5 g of phenol (Sigma-Aldrich, P1037) were then added, and 300 mL of distilled water was added. After addition of 5 g of sodium sulfite anhydrous (Daejung, 7634-4405) followed by stirring to dissolve and cooling, the final volume was adjusted to 1000 mL with distilled water and then filtered to complete the preparation.

### Example 2-2. Evaluation of Optimum Condition of Glucanase Variant

In order to evaluate the optimum pH conditions, a citrate buffer (pH 3-5), a potassium phosphate buffer (pH 6-7), a Tris-HCl buffer (pH 8-9), and a glycine-NaOH buffer (pH 10-12) were used. In addition, 50 mM sodium acetate (pH 5.2) was used as a buffer to evaluate the optimum temperature conditions, and the activity was measured at each of 40°C, 50°C, 60°C, 70°C, 75°C, and 80°C.

In order to evaluate the thermal tolerance of the glucanase variant, an appropriate amount of the enzyme was allowed to stand at 60°C, 70°C, 75°C, and 80°C for 5 minutes each, and then the activity was evaluated at 37°C.

### Example 3. Evaluation Result of Properties of Glucanase Variant

### Example 3-1. Evaluation of Optimum Condition

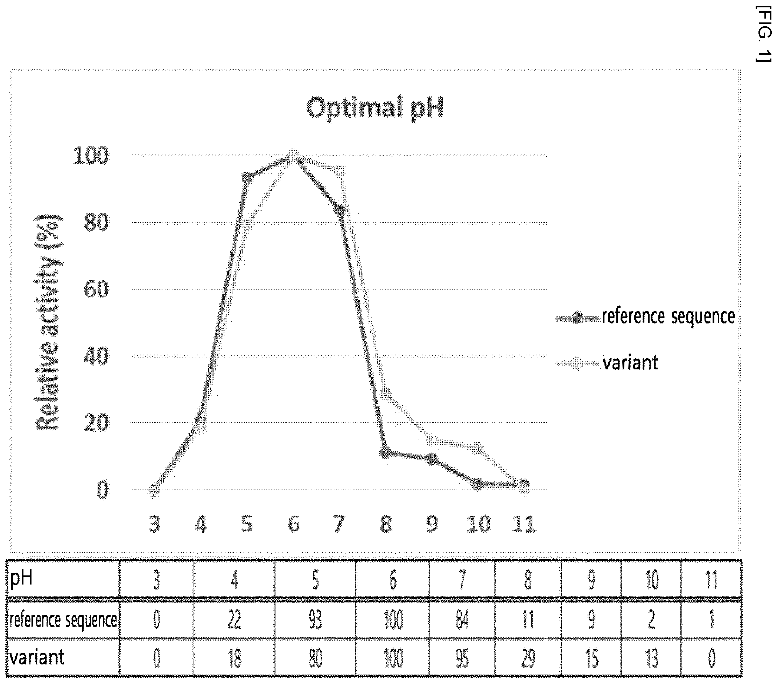

The optimum conditions for glucanase were evaluated for each temperature and pH. As a result of the evaluation, both the control group and the variant showed high relative activity at pH 5-7. The highest activity was confirmed at pH 6, and the activity was rapidly lost under alkaline conditions (FIG. 1).

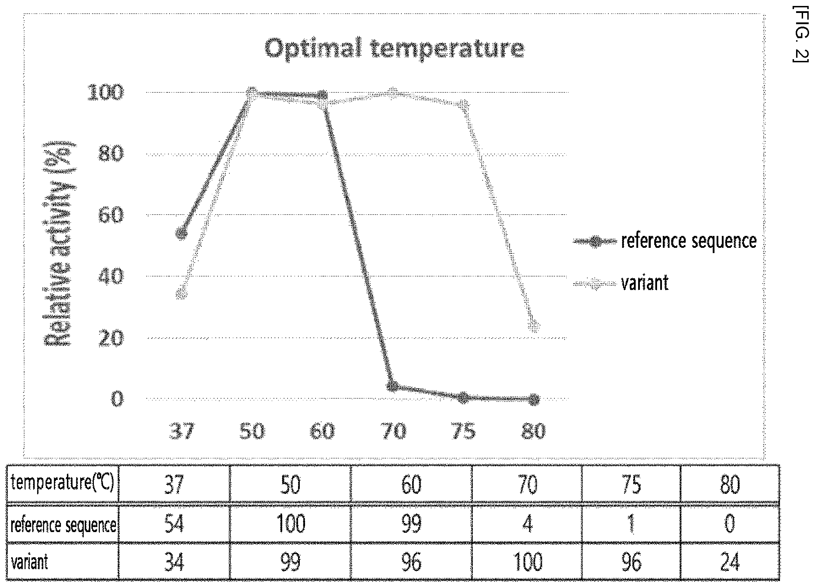

However, there was a significant difference between the two enzymes in the temperature characteristics. Although similar characteristics were observed until 60°C, at 70°C, only 4% of the activity remained in the control group, while the variant showed 100% activity. In addition, the variant showed 24% activity even at 80°C, and based on this, it was confirmed that the activity of the variant was enhanced at high temperature (FIG. 2).

### Example 3-2. Evaluation of Thermal Tolerance

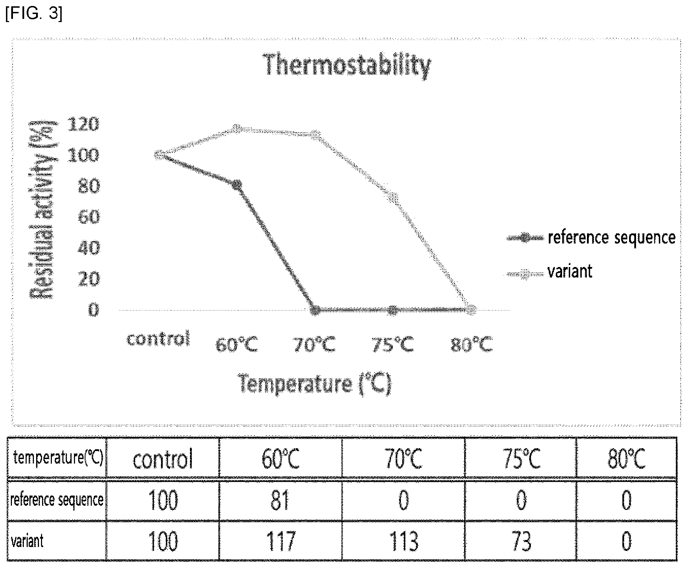

In order to evaluate the thermal tolerance of glucanase, the purified control group and variant were allowed to stand in each thermal condition from 60°C to 80°C, and then the residual activity was evaluated at 37°C, and the activity was evaluated based on the activity at 37°C (100%) (FIG. 3).

As a result, it was confirmed that 81% of the activity of the control group remained until 60°C, but all activity was lost at 70°C or higher. In contrast, it was confirmed that the variant showed 73% of the residual activity until 75°C, and there was no loss of activity until 70°C.

Base on the above, it was confirmed that the novel glucanase of the present application has improved thermal tolerance and thermal stability.

From the foregoing, a skilled person in the art to which the present application pertains will be able to understand that the present application may be embodied in other specific forms without modifying the technical concepts or essential characteristics of the present application. In this regard, the exemplary embodiments disclosed herein are only for illustrative purposes and should not be construed as limiting the scope of the present application. On the contrary, the present application is intended to cover not only the exemplary embodiments but also various alternatives, modifications, equivalents, and other embodiments that may be included within the spirit and scope of the present application as defined by the appended claims.

## Claims

1. A polypeptide of SEQ ID NO: 4, having glucanase activity.

2. The polypeptide of claim 1, wherein one or more properties of the polypeptide selected from thermal tolerance and thermal stability are improved compared to a polypeptide of SEQ ID NO: 2.

3. A polynucleotide encoding a polypeptide of SEQ ID NO: 4.

4. A microorganism, comprising one or more among a polypeptide of SEQ ID NO: 4; a polynucleotide encoding the polypeptide; and a vector containing the polynucleotide.

5. A composition for degrading glucan, comprising one or more among a polypeptide of SEQ ID NO: 4; and a microorganism comprising one or more among a polypeptide of SEQ ID NO: 4, a polynucleotide encoding the polypeptide, and a vector containing the polynucleotide.

6. A method for degrading glucan, comprising: reacting one or more among a polypeptide of SEQ ID NO: 4; and a microorganism comprising one or more among a polypeptide of SEQ ID NO: 4, a polynucleotide encoding the polypeptide, and a vector containing the polynucleotide with glucan.

7. A composition, comprising one or more among a polypeptide of SEQ ID NO: 4; and a microorganism comprising one or more among a polypeptide of SEQ ID NO: 4, a polynucleotide encoding the polypeptide, and a vector containing the polynucleotide.

8. A method for preparing feed products, comprising: mixing a feed additive composition comprising one or more among a polypeptide of SEQ ID NO: 4; and a microorganism comprising one or more among a polypeptide of SEQ ID NO: 4, a polynucleotide encoding the polypeptide, and a vector containing the polynucleotide with a feed component.

9. The composition according to claim 7, wherein the composition is for use in food, feed additive, food preparation, detergent, fabric or paper processing.

10. A method for preparing a polypeptide having glucanase activity, comprising: culturing a microorganism comprising one or more among a polypeptide of SEQ ID NO: 4; a polynucleotide encoding the polypeptide; and a vector containing the polynucleotide.
